(19) Europäisches Patentamt
European Patent Office
Office européen des brevets

(11) EP 0 947 184 A1

(12) EUROPEAN PATENT APPLICATION

(43) Date of publication:
06.10.1999 Bulletin 1999/40

(51) Int. Cl.⁶: **A61F 13/15**

(21) Application number: 98105685.6

(22) Date of filing: 28.03.1998

(84) Designated Contracting States:
**AT BE CH DE DK ES FI FR GB GR IE IT LI LU MC NL PT SE**
Designated Extension States:
**AL LT LV MK RO SI**

(71) Applicant:
**THE PROCTER & GAMBLE COMPANY
Cincinnati, Ohio 45202 (US)**

(72) Inventor: **Carlucci, Giovanni
66100 Chieti (IT)**

(74) Representative:
**Kohol, Sonia et al
Procter & Gamble
European Service GmbH
Sulzbacher Strasse 40-50
65823 Schwalbach am Taunus (DE)**

(54) **The use of a breathable absorbent article to maintain ideal skin surface temperature**

(57) The present invention relates to absorbent articles such as sanitary napkins and the use of breathable backsheet constructions therein to maintain the skin surface temperature of the wearer of said article within +/- 1°C of the skin surface temperature in the absence of said article.

EP 0 947 184 A1

## Description

Field of the Invention

The present invention relates to absorbent articles and in particular sanitary napkins and the use of breathable backsheet constructions therein to maintain the skin surface temperature at the area of use of the absorbent article within the normal skin surface temperature range.

Background of the Invention

The human body typically operates at, and maintains, an elevated temperature with respect to the environment. In addition, many of the metabolic/physiological reactions within the body are exothermic and serve to generate an additional heat load. Nevertheless, the allowable range of 'internal' temperature of the body needs to be carefully controlled to within a relatively narrow range. The body achieves this delicate temperature balance by controlling, in warmer environments, primarily the heat loss mechanisms. Transport of latent heat away from the body generally occurs via evaporative heat loss which is linked to trans epidermal heat loss (TEWL), and perspiration and to a lesser degree radiative heat exchange. In cooler environments these processes are reduced to minimise the latent heat loss from the body thus maintaining the required temperature.

In referring to body temperature it is important to differentiate between the body's core (or internal) temperature and the temperature of the surface of the skin. It is required that a normal healthy human being has a constant internal body temperature with minimal variation. This internal body temperature is maintained by a number of mechanisms such as perspiration in hot environments and shivering in cold environments. Maintenance of the internal body temperature at its ideal value, may as a result cause changes in the skin surface temperature. The skin temperature changes can either affect the entire skin surface or alternatively, the temperature changes may be localised to a particular part of the skin surface. Infact, specific regions of the body which have greater blood flow closer to the skin surface and/or having greater natural occlusion, such as present for example in the arm pit and in the genital regions, will normally tend to exhibit localised higher skin surface temperatures in comparison to the skin surface on other parts of the body.

Excluding medical factors, there are generally a number of factors that can be considered as key variables that effect the skin surface temperature at a given region of the skin. These include climatic factors, metabolic factors, physical exertion and clothing.

In hot and/or humid conditions the passive heat loss mechanisms on the skin surface are less efficient, resulting in a higher skin surface temperature and additional heat loss through perspiration. Conversely, in a cooler environment, a cooler skin surface is typically observed. For example a person moving from an outside hot summer's day environment to inside into a cool house, can result in up to a 6°C skin surface temperature fluctuation over a relatively short time frame.

Metabolic factors also affect the skin surface temperature. The body performs certain cycles of metabolic or physiological state activity that result in changes in the amount of heat that the body generates. For example, after eating, the level of heat generated by the body rises and a corresponding increase in skin temperature due to higher heat transport to the surface of the skin can occur. Similarly, stress factors can also affect the overall metabolic/physiological activity levels. This results in a higher heat load to be dissipated. In addition stress also initiates sweat gland activity (perspiration) which can alter the skin surface temperature balance within a short time frame.

Another skin surface temperature factor is physical activity. Physical exertion will also result in a higher heat generation than normal, that will alter the overall heat management status of the body and effect the skin surface temperature.

Finally, clothing is also a key factor that affects the heat exchange rate of the skin surface. This can be either through thermal insulation or through reduced moisture exchange that traps the latent heat close to the body. For example, wearing tight fitting clothing causes heat to be trapped close to the body in the region covered by the clothing resulting in a temperature rise of the surface of the skin. This is due to restricted moisture flow away from the body and due to the thermal insulation properties of the material from which the clothing is manufactured.

The detection by the human body of such temperature changes on the skin surface is achieved by thermal sensors within the skin. These sensors are however most sensitive to particular types of temperature changes.

For example the body more readily detects rapid temperature changes compared to slow temperature changes. Similarly, the body more easily recognises external temperature changes on the skin surface rather than temperature changes internally. Likewise temperature changes on a localised area of the skin surface are more readily detected than changes that occur over the whole surface; certain areas of the body being more sensitive than other areas, such as the fingers for example. The degree of sensitivity to temperature change is believed to be linked to the density of thermal sensors at particular sites of the body. Hence, as the skin surface temperature fluctuates to maintain the required internal body temperature, a temperature change of several degrees centigrade uniformly applied over the entire skin surface, over a relatively long period of time, may not even be perceived. On the contrary, a rapid temperature change due to an external factor between the left and right hand or finger tip of only 1°C, is readily detectable by the body.

The fluctuation in skin surface temperature, particularly at elevated temperatures is typically considered as an uncomfortable status for the body. Such skin surface temperature variations are yet further exacerbated by other external factors. In particular, the presence of an absorbent article such as a diaper, sanitary napkin, incontinence product or perspiration pad placed directly in contact with or juxtaposed against the surface of the skin will also further affect the skin surface temperature status. For the duration of the period of wear of such articles all of the previously mentioned factors will of course contribute to the temperature of the skin surface over the area of the skin upon which the article is placed. Typically, the overall result of utilising such an article is a localised skin surface temperature rise at the area of the skin surface covered by the product.

For example, wearing a sanitary napkin in the genital area typically results in a high degree of localised occlusion. This disrupts the evaporative flow of moisture from this region and thus restricts the latent heat transfer. Thereby heat is caused to be trapped close to the body in the region covered by the napkin. In addition, thermal insulation properties of the components typically present in such pads further prevents heat dissipation. The skin surface temperature where the napkin is located hence rises.

Typically, the reference for the evaluation of 'normal' temperature in the genital region is the skin surface temperature achieved whilst wearing a panty. Experimental analysis has determined that the average skin surface temperature whilst wearing a sanitary napkin can rise by more than 1.2 °C above the skin surface temperature obtained on the same wearer, in the absence of a sanitary napkin. At such elevated temperatures, a clear perception of discomfort described as feeling hot and sweaty in this area is detected. In fact, feeling hot or a perception of heat discomfort in the genital region whilst wearing a sanitary napkin is a very common occurrence which is more extreme in warmer/humid climates, but nevertheless also clearly present in cooler, drier climates.

Thus, an absorbent article that is able to maintain a more comfortable skin surface temperature over the skin surface which it covers, that is closer to the temperature of the skin surface when the consumer is not wearing such an article, will provide a more ideal or 'normal' temperature perception in the genital region and is hence highly desirable.

It has now been surprisingly found that this problem can be addressed by the use of a moisture vapour permeable liquid impervious backsheet, preferably having a moisture vapour transport rate of at least $800g/m^2/24hrs.$ within an absorbent article, which thereby ensures that the temperature variation due to the presence of the absorbent article is not greater than 1°C.

### Summary of the Invention

The present invention relates to the use of a breathable absorbent article in particular an absorbent article having a moisture vapour permeable, liquid impermeable backsheet. Said article comprises a wearer facing surface and a garment facing surface and said backsheet comprises said garment facing surface. Accordingly, the present invention relates to the use of said backsheet to maintain the average skin surface temperature of the wearer of said article at the common interface between said article and said skin surface at a temperature of from +1°C to -1°C of the temperature of said skin surface at said common interface in the absence of said article.

### Detailed Description of the Invention

The present invention relates to absorbent disposable articles such as sanitary napkins, panty liners, incontinence products, perspiration pads and baby diapers. According to the present invention these products comprise a wearer facing surface, and a garment facing surface. Typically, such products comprise a liquid pervious topsheet providing the wearer facing surface, a backsheet providing the garment facing surface and an absorbent core intermediate said topsheet and said backsheet. The present invention relates to such articles which comprise a moisture vapour permeable, liquid impervious backsheet more commonly referred to as a breathable backsheet.

The absorbent articles can also comprise any of the components or features usual in the art, in particular side wrapping elements, side flap components, or wings as well as any sort of extensibility or elastication feature can be comprised in absorbent articles. For example a typical sanitary napkin or panty liner comprises an adhesive area on the garment facing surface of the backsheet providing the panty-fastening adhesive which is covered by a release paper, wrapper or the like prior to the use of the article.

The absorbent article for absorbing liquid is described below by reference to a sanitary napkin or panty liner. However products such as adult or baby diapers, incontinence products or perspiration pads can similarly benefit from the present invention.

Backsheet

The absorbent article according to the present invention comprises as an essential feature a breathable backsheet. The backsheet primarily prevents the extrudes absorbed and contained in the absorbent structure from wetting articles that contact the absorbent product such as underpants, pants, pyjamas and undergarments thereby acting as a barrier to fluid transport. The backsheet typically extends across the whole of the absorbent structure and can extend into and form part of, or all of the sideflaps, side wrapping elements or wings. However, in addition to acting as a liquid barrier, the breathable backsheet of the present invention permits the transfer of at least moisture vapour, preferably both vapour and air through it and thus allows the circulation of gases into and out of the backsheet.

It has now been further surprisingly identified that the utilisation of a breathable backsheet also delivers desirable benefits to the skin surface of the wearer which is adjacent to the absorbent article in use. In use the absorbent article is typically positioned directly in contact with or juxtaposed against an area of skin surface of the wearer such as the genital region or in the arm pit. Consequently, a common interface between the absorbent article and an area of skin surface is thereby provided. Unexpectedly, the use of a breathable backsheet maintains the average skin surface temperature of the wearer of said article at the common interface between the article and the skin surface at an average temperature of from +1°C to -1°C of the temperature of said skin surface at said common interface in the absence of said article. Preferably the average skin surface temperature is maintained within +0.5°C to -0.5°C of the temperature of said skin surface at said common interface in the absence of said article and most preferably the average skin surface temperature is maintained at the same temperature as the temperature of said skin surface at said common interface in the absence of said article.

The skin surface temperature as referred to herein is the average temperature of the skin surface, at the skin surface to air interface and is measured according to the test method described hereinafter.

According to the present invention breathable backsheets suitable to provide the benefits as described herein above may be any breathable backsheet known in the art comprising at least one moisture vapour permeable layer. Suitable moisture vapour permeable layers include 2 dimensional, planar micro and macro-porous films; macroscopically expanded films; formed apertured films; monolithic films and nonwoven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional porous planar layers of the backsheet may be made of any material known in the art, but are preferably manufactured from commonly available polymeric materials. Suitable materials are for example Goretex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane™ available from BF Goodrich (Belgium).

Preferred breathable backsheets for use herein are those having a high moisture vapour exchange, most preferably both a high moisture vapour and high air exchange. Particularly preferred backsheets for the present invention comprise at least two layers comprising at least one layer selected from the above, such as a microporous layer or an apertured formed film and an additional layer which may also be selected from the above listed backsheets. The most preferred breathable backsheet component comprises a microporous film and an apertured formed film; or a microporous film and a hydrophobic fibrous layer; or an apertured formed film and a hydrophobic fibrous layer. Preferably, the hydrophobic fibrous layers are hydrophobic non woven.

Preferably the backhseet of the present invention has a moisture vapour transfer rate of at least 200g/m$^2$/24hrs, preferably from 600 g/m$^2$/24hrs. to 2200 g/m$^2$/24hrs and most preferably from 800 g/m$^2$/24hrs. to 2200 g/m$^2$/24hrs as measured in the test described herein after in test methods. Furthermore, the backsheet of the present invention in addition to allowing the flow of bodily moisture through it also preferably allows the circulation of air in and out of the backsheet.

The topsheet

According to the present invention any of the typically utilised topsheets in absorbent articles can be employed herein.

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; and thermoplastic scrims and combinations thereof. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers and are preferably hydrophobic.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures. Particularly useful embodiments of such hybrid topsheets are disclosed in PCT publications WO 93/09744; WO 93/11725 or WO 93/11726 and US 4 780 352.

When referring to the topsheet a multi layer structure or a mono layer structure is contemplated. The hybrid topsheet mentioned above is such a multi layer design but other multi layer topsheets such as primary and secondary topsheet designs are also considered.

The topsheet typically extends across the whole of the absorbent structure and outside the area coextensive with the absorbent structure. The topsheet can extend and form part or all of the preferred side flaps, side wrapping elements or wings.

Absorbent core

According to the present invention the absorbent cores suitable for use in herein may be selected from any of the absorbent cores or core system known in the art. As used herein the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents.

A preferred sanitary napkin or panty liner made according to the present invention has a pair of side wrapping elements or "undergarment covering components". These elements or components provide coverage of the wearer's panties to reduce side soiling (i.e., staining of the edges of the panty crotch) and are typically smaller than conventional flaps or wings.

The function of the side wrapping elements, whether integral with the article or joined to the article after being formed separately, is further improved by rendering them extensible in one or both directions parallel to the longitudinal axis and/or lateral axis. The extensibility can be provided across all or only part of the side wrapping elements and can be achieved by pleating or ring-rolling those parts which are to be rendered extensible.

According to the present invention the topsheet, backsheet and absorbent core components are joined together to provide the absorbent article. Typically, at least two, preferably all of the components of the article are joined to form the article. Each of said components of the absorbent article comprise at least one layer and have a wearer facing surface and a garment facing surface. Typically, adjacent garment facing surfaces form a common interface with the wearer facing surface of an adjacent component or layer. The elements or layers are joined together across this common interface. In this manner, the topsheet is joined to the absorbent core, and the core is joined to the backsheet. In addition, the topsheet may be directly or indirectly by joined to the backsheet at the periphery of the absorbent article. Furthermore, particularly in sanitary napkin, panty liner and incontinence product applications, the garment facing surface of the backsheet also provides the surface to which the absorbent article is releasably joined to the garment of the user of the product. Prior to use, this surface is typically provided with a protective cover. Any means known in the art to join the components of the absorbent article and provide the garment fastening. May be utilised such as utilising a continuous layer of adhesive, a patterned layer of adhesive, such as spirals, or spots, or using heat bonds, pressure bonds, mechanical bonds and the like.

According to the present invention the absorbent article itself preferably has a moisture vapour permeability of at least 200 $g/m^2/24hrs$, preferably at least 400 $g/m^2/24hrs$, most preferably at least 600 $g/m^2/24hrs$.

## Test methods

Moisture vapour permeability test

The basic principle of the test is to quantify the extent of water vapour transmission of a backsheet construction and an absorbent article. The test method that is applied is based on a standardized textile industry applied test method and commonly referred to as the "cup test method". The test is performed in a stable temperature/humidity laboratory maintained at a temperature of 23° C at 50% RH for a period of 24 hours.

**Apparatus:**

    1) Sample cup of open area = 0.00059 $m^2$)
    2) Syringe to introduce the distilled water into the completed sample cup.
    3) Wax to seal the cup once sample has been arranged.
    4) A circular punch to facilitate preparation circular samples of diameter = 30 mm.
    5) Laboratory of stable climatic conditions (23° C ± 0.5°C / 50% RH ± 1 % RH)
    6) Laboratory balance accurate to 4 decimal places.

Sample Preparation / Measurements:

The test is be performed on the absorbent article product or the backsheet construction. A representative article is selected and a sample is cut to size using the punch. The sample cut is sufficiently large to adequately overlap the sample holder and to ensure material that may have been damaged or undesirably stretched due to the cutting operation lies outside of the measurement centre when the measurement is performed. The sample is so arranged onto of the sample cup so as to fully overlap the cup. The sample is oriented so as to ensure that the surface exposed to the laboratory environment is the same that would be found while wearing the article.

The closure ring of the sample cup is then placed onto the sample and pushed down. This ensures that the excess material is held firmly in place and does not interfere with the measurement. A wax is then applied to the entire surface of the closure ring to ensure the whole upper part of the apparatus is closed to the environment. Distilled water (5 ± 0.25 ml) is introduced with the syringe into the sealed sample cup via the minute perforation. Finally this perforation is sealed with silicone grease.

The entire cup (containing sample and water) is weighed and the weight recorded to 4 decimal places. The cup is then placed in a ventilation stream generated by a fan. The air flowing over the top of the sample cup is 3 ±0.3 m/sec and confirmed via a wind velocity meter ("Anemo", supplied by Deuta SpA., Italy). The sample cup remains in the ventilated test field for a period of 24 hrs and is then re-weighted. During this period if the test sample is sufficiently breathable the liquid in the sample holder is able to diffuse out of the sample holder and into the laboratory environment. This results in a reduction in the weight of water in the sample holder that can be quantified on re-weighing the complete sample cup following the 24 hr period. The vapour permeability value is determined as the weight loss divided by the open area of the sample holder and quoted per day.

$$\text{i.e. Vapour Permeability} = \text{Weight Loss (g)} / (0.00059 \, m^2 / \, 24 \, hrs.)$$

**Skin Temperature Measurement**

The preferred approach to assess temperature in the region, under a pad, is to use a thermal imagery system able to differentiate relatively small temperature differences (resolution 0.1°C). This enables the temperature profile under the pad to be assessed and is less influenced by localized variability that may result from irritation caused by sensors at such an imitate body site.

An IR imaging system supplied by AMEGA of Sweden is used to take the temperature measurements. The IR camera (Thermovision 550) operates over a wavelength of 2.5 - 5.6 micrometers and has a resolution of 72,800 pixels, delivering 0.1° C temperature resolution. It is set up and used according to the manufacturers instructions.

The protocol used closely reflects a normal wearing situation. A group of 10 women wearing their normal panties and a hospital gown are required to remain in a temperature and humidity controlled environment (25°C/50%RH) for a period of 3 hours. In this time they perform a variety of tasks, such as sitting, standing and walking without strenuous activity, regularly spaced over the 3 hr period. After completion of the 3 hrs. the woman is seated before the camera and an image of the region is recorded. The panties are removed and at precisely 30 second later a second image, recording the thermal profile of the skin is taken. The woman then places the test pad in the panty and the procedure is repeated. After 3 hrs the pad in panty image is recorded and on removal of the pad/panty, after exactly 30 seconds, an image of the skin covered by the pad is recorded. The images are stored in the camera memory and are downloaded to a computer for data elaboration at a later stage. The data elaboration is performed using the software supplied with the *camera* (IR WIN 5.1). This allows an average temperature profile of the skin or exposed surface to be elaborated and documented along with the stored images.

**Results**

Examples representative of the present invention are given below. Each example was derived from a sanitary napkins produced under the name of "Always Ultra Normal" available form Procter and Gamble GmbH, Schwalbach/Germany which had been manufactured according to normal manufacturing procedures and then modified as described below

Product A is the currently marketed Europe "Always Ultra Normal" in Europe that features a backsheet material that is not breathable.

Product B is identical to Product A wherein the backsheet has been replaced by a 2 layer backsheet construction. The first layer backsheet that is placed directly in contact with the absorbent tissue core is a formed apertured film backsheet layer that is a blend of low and high density PE with a crush resistant hexagonal hole configuration {supplied by Tredegar Film Products B.V. Holland under the manufacturing code AS 225 HD 25}. The second and final layer is a microporous film supplied by Exxon Chemical Company USA under the manufacturing code Exxaire XBF-112W.

Each of the products A and B were tested by ten testers. The products were tedsted dry and loaded with 3ml of distilled water warmed to body temperature and applied to the centre of the pad. The average temperature of the skin surface as defined herein was measured and is given below.

|  | Temp vs. Panty (°C) | WVTR g/m$^2$.24hrs. (Backsheet) | WVTR g/m$^2$.24hrs (Product) |
|---|---|---|---|
| Product A | >1.2 | 0 | zero |
| Product B | <1.0 | 950 | 660 |

Claims

1. The use of a moisture vapour permeable, liquid impervious backsheet in an absorbent article, to maintain the average skin surface temperature of the wearer of said article at the common interface between said article and said skin surface at a temperature of from +1°C to -1°C of the temperature of said skin surface at said common interface in the absence of said article.

2. The use according to claim 1, wherein said average skin temperature is maintained within +0.5°C to -0.5°C of the temperature of said skin surface at said common interface in the absence of said article.

3. The use according to claim 1, wherein said average skin temperature is maintained at the same temperature as the temperature of said skin surface at said common interface in the absence of said article.

4. The use according to claim 1, wherein said backsheet has a water vapour transfer rate of at least 200g/m$^2$/24hrs.

5. The use according to claim 4, wherein said backsheet has a moisture vapour transfer rate of from 600 g/m$^2$/24hrs. to 2200 g/m$^2$/24hrs.

6. The use according to claim 4, wherein said backsheet has a moisture vapour transfer rate of from 800 g/m$^2$/24hrs. to 2200 g/m$^2$/24hrs.

7. The use according to any one of the preceding claims, wherein said absorbent article has a moisture vapour permeability of at least 200 g/m$^2$/24hrs.

8. The use according to any one of the preceding claims, wherein said backsheet comprises at least one layer selected from apertured polymeric formed films, 2-dimensional planar apertured films, monolithic films or nonwovens.

9. The use according to claim 8, wherein said backsheet comprises at least two layers, and wherein both of said layers are independently selected from apertured formed polymeric films and 2-dimensional planar apertured films.

10. The use according to claim 8, wherein said backsheet comprises at least two layers, a first layer comprising an apertured layer and a second layer comprising a fibrous layer.

11. The use according to any one of the preceding claims, wherein said absorbent article is a sanitary napkin or a panty liner.

## EUROPEAN SEARCH REPORT

**European Patent Office**

**Application Number**

EP 98 10 5685

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP 0 813 849 A (PROCTER & GAMBLE) 29 December 1997<br>* page 3, line 48 - line 49; examples; tables *<br>* page 4, line 26 - page 5, line 10 *<br>--- | 1,4-11 | A61F13/15 |
| A | GB 2 171 016 A (KAO CORP) 20 August 1986<br><br>* page 4, line 5 - line 49; tables 6-8 *<br>* page 5, line 54 - line 60 *<br>--- | 1,4,5,7,8,11 | |
| A | EP 0 640 328 A (KAO CORP) 1 March 1995<br><br>* page 3, line 20 - line 23 *<br>* page 3, line 47 - line 52 *<br>* page 5, line 33 - line 50; claim 5; table 1 *<br>--- | 1,4,5,7,8,11 | |
| A | EP 0 307 116 A (MITSUBISHI KASEI VINYL ;MITSUBISHI CHEM IND (JP)) 15 March 1989<br>* page 2, line 43 - line 50; tables 7,8,11,12 *<br>* page 3, line 12 - line 17 *<br>* page 5, line 18 - line 38 *<br>--- | 1,4,5,7,8,11 | TECHNICAL FIELDS SEARCHED (Int.Cl.6)<br><br>A61F |
| A | WO 95 27516 A (KAO CORP ;SUZUKI MIKIO (JP); TAKEUCHI KEN (JP); WADA MASASHI (JP)) 19 October 1995<br>* page 1, line 10 - line 13; table 2 *<br>* page 13, line 6 - line 9; claim 1 *<br>--- | 1,4,5,7,8,11 | |
| A | WO 97 30671 A (PROCTER & GAMBLE) 28 August 1997<br>* page 3, line 23 - page 4, line 2 *<br>* page 14, line 30 - page 15, line 5 *<br>* page 33, line 17 - line 22 *<br>* page 38, line 28 - page 39, line 22 *<br>--- | 1,8-10 | |

-/--

The present search report has been drawn up for all claims

| Place of search | Date of completion of the search | Examiner |
|---|---|---|
| THE HAGUE | 18 September 1998 | Mirza, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 98 10 5685

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|----------|------------------------------------------------------------------------------|-------------------|---------------------------------------------|
| A | EP 0 806 195 A (PROCTER & GAMBLE) 12 November 1997 * column 2, line 29 - line 49; claims 1,6,7,15 * * column 9, line 10 - column 10, line 51 * * column 19, line 7 - line 12 * | 1,8,10, 11 | |

TECHNICAL FIELDS SEARCHED (Int.Cl.6)

The present search report has been drawn up for all claims

| Place of search | Date of completion of the search | Examiner |
|-----------------|----------------------------------|----------|
| THE HAGUE | 18 September 1998 | Mirza, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)